# EUROPEAN PATENT APPLICATION

(11) **EP 4 717 761 A2**
(43) Date of publication of application: **01.04.2026**
(21) Application number: 25205876.3
(22) Date of filing: 30.09.2025
(51) Int. Cl.: C12N 1/066, A61K 8/49, C12M 1/00, C12P 17/18, C12P 23/00, C12P 25/00, D06P 1/34

(54) **METHOD FOR BIOPIGMENTS EXTRACTION FROM MICROBIAL CULTURES**

(30) Priority: 30.09.2024 PT 2024119728
(71) Applicant: Kod-Bio Lda, 4455-533 Perafita (PT)
(72) Inventor: LEAL EUSÉBIO, NÁDIA CRISTINA, 4455-533 PERAFITA (PT); FIGUEIRINHAS COSTA, RICARDO GALAMBA, 4455-533 PERAFITA (PT)
(74) Representative: Patentree

(57) **Abstract**

The present disclosure relates to an apparatus and method for obtaining a biopigment extract from a microorganism, and to the biopigment extract obtained by said method. Furthermore, the present disclosure relates to articles or products comprising said biopigment extract.

## Description

### TECHNICAL FIELD

The present disclosure relates to the field of biopigment extraction, and more specifically to a method for obtaining a biopigment extract from a microorganism, using a combination of mechanical cell disruption and sequential filtration steps to obtain a biopigment extract with enhanced purity and consistency. Furthermore, the present disclosure relates to the biopigment extract obtained by said method, and to products and articles comprising said biopigment extract, in particular industrial products, for instance, textile articles.

### BACKGROUND

Bio-based dyes are becoming a growing alternative to traditional petrol dyes. Bio-based dyes are derived from natural and renewable sources such as plants, insects, or even microorganisms (Muthusamy et al., 2020). They offer several advantages over traditional dyes, including environmental sustainability, reduced toxicity, and potential health benefits. These dyes can also give the fabric finishing properties else achieved by synthetic compounds. Microbial dyes hold the potential to be a new revolution in the field of dyeing since they are non-toxic and cheaper to produce, compared to traditional dyes (Aman Mohammadi et al., 2022).

This innovative approach offers several advantages and aligns with the growing demand for sustainable and eco-friendly practices in the industry. Scientists are continuously exploring new microorganisms and application techniques to improve color range of microbial dyeing.

Natural pigments are products produced by the secondary metabolism of bacteria or fungi. Some examples of families of microbial pigments include (i) carotenoids: these are red, yellow, and orange pigments that are commonly produced by bacteria and algae; they play a vital role in photosynthesis and act as antioxidants, protecting cells from oxidative damage (Baskar et al., 2010); (ii) melanin: these are brown or black pigments that are produced by bacteria, fungi, and some animals; they protect cells from UV radiation, oxidative damage, and help to resist antibiotics (Kamarudheen et al., 2019); (iii) flavonoids: these are yellow pigments produced by bacteria and fungi; they function as coenzymes in metabolic pathways and as antioxidants (Hozzein et al., 2021); (iv) indigoids: these are blue pigments that are produced by bacteria, fungi, and plants; they have antimicrobial properties and can inhibit the growth of some bacterial species (Fabara & Fraaije, 2020).

The concept of using biopigments sourced from microbial origin for textile dyeing is not a new idea and has gained traction within the textile industry, especially in the last five years. Several companies have already made strides in this direction, showcasing the viability of biobased pigments for sustainable textile coloration. These companies have harnessed the capabilities of microorganisms to produce pigments and applied them to fabrics, aligning with the demand for eco-friendly alternatives to conventional synthetic dyes. However, these companies are limited to a small set of colors and low color fastness (e.g. low resistance to UV light, washing and rubbing), hindering their successful widespread introduction in the textile industry. Moreover, these companies are often limited to specific strains of microorganisms or particular types of biopigments that can be efficiently extracted using their current methods. Existing processes typically rely on either chemical extraction techniques, which generally involve the use of organic solvents and harsh reagents, or enzymatic methods that require precise control over conditions and can be costly at scale. Additionally, mechanical cell disruption methods currently employed are not fully optimized for a broad range of microbial cultures, leading to suboptimal yields and inconsistencies in pigment quality.

There is a clear need for a novel, more versatile method that can efficiently extract a wide variety of biopigments from diverse microbial sources.

These facts are disclosed in order to illustrate the technical problem addressed by the present disclosure.

### GENERAL DESCRIPTION

The present disclosure relates to a novel method for extracting biopigments from microorganisms, utilizing a mechanical press system for cell disruption, in particular a French press system, followed by a sequential filtration and purification process. This integrated approach allowed to optimize yield, purity, and scalability of the extraction process, offering a more efficient alternative to existing methods.

The present disclosure relates to an integrated approach that combines mechanical cell disruption, such as the use of a French press, with sequential filtration steps tailored to different microbial cultures. Surprisingly, this method not only improves the efficiency of pigment extraction but also enhance the purity and consistency of the final product. Additionally, the method of the present disclosure does not use organic solvents.

By optimizing mechanical disruption techniques for various microorganisms and coupling them with a carefully designed filtration process, the method of the present disclosure significantly expands the range of usable biopigments. This allows the production of a broader spectrum of colours and shades, catering to diverse textile applications while maintaining the environmental benefits associated with biobased pigments. Furthermore, it reduces reliance on chemical solvents and minimize energy consumption, aligning with the industry's sustainability goals.

When the biopigment extract obtained with the method of the present disclosure was applied to different textile substrates, the resulting coloration exhibited significantly more diversified RGB values compared to fabrics treated with extracts obtained with other methods described in the prior art. In particular, fabrics dyed with extracts from the comparative method showed similar and consistently high RGB values, typically in the range of beige or light yellow/grey shades, evidencing limited pigment release.

Surprisingly, fabrics dyed with extracts prepared according to the method of the present disclosure displayed markedly lower and variable RGB values, corresponding to darker and more intense colours, across a wide range of textile materials and a wide range of microorganisms. This demonstrates that the method of the present disclosure improves the release of intracellular pigments that otherwise remain unavailable, thereby providing enhanced dyeing performance and expanding the achievable colour palette.

The present disclosure relates to a method for obtaining a biopigment extract from a microorganism, comprising:
providing a microbiological culture suspension;
subjecting the microbiological culture suspension to at least a cycle of mechanical disruption using a French press, at a pressure ranging from 13.7895 - 344.7378 MPa (2,000 to 50,000 psi);
performing a first filtration of the disrupted cell mixture through a first filter,
wherein said first filter has a pore size ranging from 0.45 to 15 micrometers;
optionally performing a second filtration of the filtrate obtained from the first filtration through a second filter, wherein said second filter has a pore size ranging from 0.2 to 0.45 micrometers,
thereby obtaining a biopigment extract.

Surprisingly the disclosed method in terms of colour performance is therefore twofold: to maximize pigment release from the biological source, ensuring a fuller expression of chromatic diversity, and to achieve fabric pigment with deeper, more intense, and visually appealing coloration, thereby enhancing both aesthetic value and functional application potential in fabric finishing (woven and non-woven finishing process).

The method of the present disclosure offers several advantages over conventional biopigment extraction techniques:
Improved Efficiency: The use of a French press at the specific pressure ranges provided, ensures complete cell disruption, leading to higher biopigment yield.
Enhanced Purity: The sequential filtration steps effectively remove cellular debris and contaminants, resulting in a biopigment extract with enhanced purity.
Consistency: The method provides a consistent process for biopigment extraction,
ensuring reproducibility and uniformity of the final product.

Sustainability: The method does not use organic solvents.

In an embodiment, the method is an organic solvent free method.

In an embodiment, the mechanical disruption using a French press is performed at a pressure from 13.7895 - 344.7378 MPa (2,000 to 50,000 psi); preferably 103.4213 - 172.3689 MPa (15,000 - 25,000 psi); more preferably 137.8951 MPa (20,000 psi).

In an embodiment, the first filter has a pore size ranging from 0.45 to 15 micrometers; preferably 0.45 micrometer to 5 micrometres; even more preferably 5 micrometers.

In an embodiment, the second filter has a pore size of ranging from 0.20 to 0.45 micrometers; preferably 0.45 micrometers.

In an embodiment, the mechanical disruption is performed at a temperature ranging from 0 °C to 40 °C; preferably 20 °C to 30 °C; more preferably 30°C.

In an embodiment, the microbiological culture suspension has a volume ranging from 10 mL - 100 L; preferably 20-40 mL; even more preferably 30 mL.

In an embodiment, the microbiological culture suspension has an optical density at 600 nm (OD600) ranging from 0.1 - 20; preferably 0.1-4; more preferably 0.5-2 (measured at 30°C using a standard UV-Vis spectrophotometer).

In an embodiment, the pH of the microbiological culture suspension ranges from 3 - 10; preferably 6-8 (measured at 30°C).

In an embodiment, the microorganism is selected from a microorganism capable of producing intracellular pigments selected from the list consisting of: bacteria, microalgae, fungi, yeast, or mixtures thereof.

In an embodiment, the microorganism is selected from the group consisting of yeast, actinobacteria and bacteria. Preferably, the microorganism is selected from the group consisting of *Pseudomonas spp., Streptomyces spp., Candida spp., Bacillus spp.,* or mixtures thereof.

In an embodiment, the microorganism is selected from the group consisting of: *Candida albicans, Streptomyces coelicolor, Bacillus subtilis, Streptomyces brasiliensis, Streptomyces griseus, Rhodotorula minuta, Bacillus nakamurai, Pseudomonas azotoformans, Pseudomonas fluorescens,* or mixtures thereof.

In an embodiment, the microorganism is selected from the group consisting of: *Streptomyces coelicolor, Streptomyces brasiliensis, Streptomyces griseus,* or mixtures thereof.

In an embodiment, the microorganism is selected from the group consisting of: *Pseudomonas azotoformans, Pseudomonas fluorescens.*

In an embodiment, the microorganism is selected from the group consisting of: *Bacillus subtilis, Bacillus nakamurai,* or mixtures thereof.

In an embodiment, the microorganism is *Candida albicans.*

In an embodiment, the microorganism is *Rhodotorula minuta.*

In a preferred embodiment, the microorganism is selected from *Streptomyces brasiliensis, Streptomyces griseus, Rhodotorula minuta* or *Bacillus nakamurai.*

In an embodiment, the mechanical disruption is performed for two or more mechanical disruption cycles; preferably for three or more mechanical disruption cycles; more preferably 3-4 cycles of mechanical disruption.

In a preferred embodiment, the mechanical disruption is performed for three disruption cycles.

In an embodiment, the method further comprises a step of drying the biopigment extract; preferably through freeze-drying.

In an embodiment, the method further comprises a step of formulating the biopigment extract into a powder, paste, or liquid concentrate.

Another aspect of the present disclosure relates to a biopigment extract obtainable by the method herein described.

Another aspect of the present disclosure relates to a textile dying method, comprising the step of applying the biopigment extract herein described to a textile substrate.

Another aspect of the present disclosure relates to a fabric or textile material comprising the biopigment extract herein described. Preferably, the fabric or textile material is selected from the list consisting of: acetate, cotton, wool, nylon, polyester, acrylic, or mixtures thereof.

Another aspect of the present disclosure relates to an article or product comprising the biopigment extract herein described. Preferably, said article/product is a textile article, fabric article, or non-woven fabric article.

In an embodiment, the article or product is a paper/cellulose-based article or product, or a plastic-based article or product.

Another aspect of the present disclosure relates to a cosmetic composition comprising the biopigment extract herein described.

Another aspect of the present disclosure relates to the use of the biopigment extract herein described as a textile dye or cosmetic dye; preferably as a cosmetic dye.

Another aspect of the present disclosure relates to an apparatus for obtaining a biopigment extract from a microorganism, comprising:
a mechanical cell disruption unit equipped with a French press capable of applying a pressure ranging from 13.7895- 344.7378 MPa (2,000 to 50,000 psi) to a microbial cell suspension;
a first filtration unit configured to receive the disrupted cell mixture from the mechanical cell disruption unit, said first filtration unit comprising a filter with a pore size ranging from 0.45 to 15 micrometers;
optionally, a second filtration unit configured to receive the filtrate from the first filtration unit, said second filtration unit comprising a filter with a pore size ranging from 0.2 to 0.45 micrometers;
a collection unit for receiving the biopigment extract obtained from the first filtration unit or from the second filtration unit.

### BRIEF DESCRIPTION OF THE DRAWINGS

The following figures provide preferred embodiments for illustrating the disclosure and should not be seen as limiting the scope of invention.

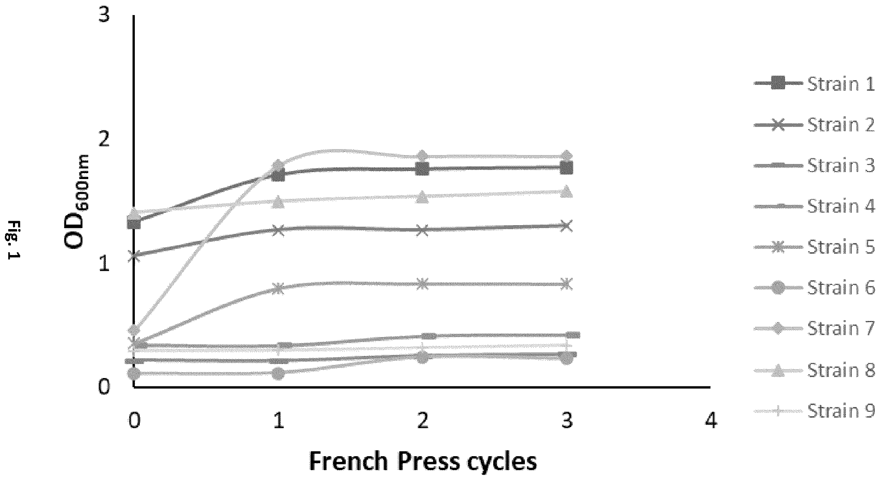

**Figure 1****:** OD (optical density) obtained for each strain before cell disruption and after each cycle. Three cycles of French Press were enough to lyse all the cells Strain 1 - 540 nm; Strain 2 - 520 nm; Strain 3 - 440 nm; Strain 4 - 560 nm; Strain 5 - 560 nm; Strain 6 - 500 nm; Strain 7 - 520 nm.

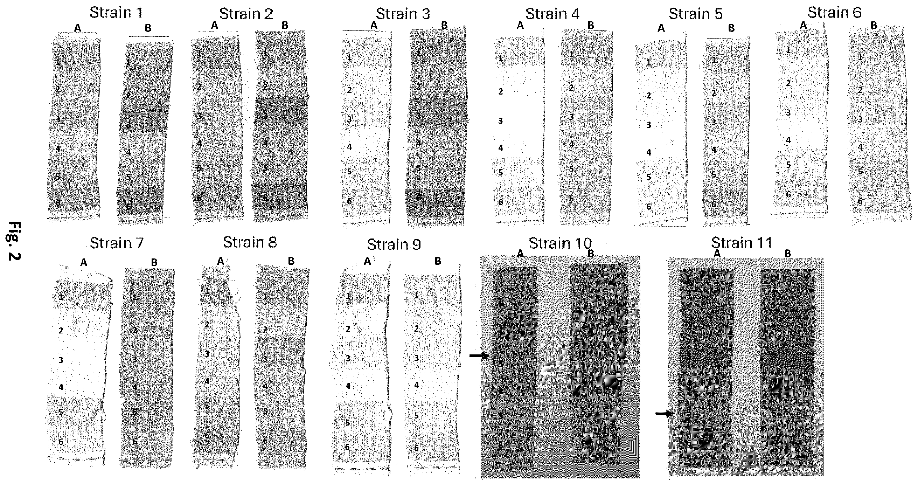

**Figure 2****:** Multifiber fabrics dyed with cultures before **(A)** and after **(B)** cell disruption. Each multifiber are composed of specific sections of Acetate (1), Cotton (2), Nylon (3), Polyester (4), Acrylic (5) and Wool (6), from top to bottom. Eleven strains were tested, nine strains for colour and 2 strains for fluorescence. For each strain are presented 2 fabrics - on the left are the fabrics coloured with biopigment extracts obtained through a comparative method (A) and on the right are the ones coloured with biopigment extracts obtained with the method of the present disclosure (B). The results were visible both for colour and fluorescence.

### DETAILED DESCRIPTION

The present disclosure relates to an apparatus and method for obtaining a biopigment extract from a microorganism, and to the biopigment extract obtained by said method. Furthermore, the present disclosure relates to articles or products comprising said biopigment extract.

The "French press cell press", or "French press", described by French and Milner (C. S. French, Harold W. Milner. [9] Disintegration of bacteria and small particles by high-pressure extrusion, (Academic Press, v.null, n.null, p.64, 1955, DOI: https://doi.org/10.1016/0076-6879(55)01013-6) is a piece of equipment used in laboratories to disrupt cell walls and cell membranes, used to obtain enzymes and DNA. The French press consists of a hydraulic pump that drives a piston. The piston forces the liquid sample through a tiny valve under high pressure. As the sample passes through the valve, the cells experience shear stress, resulting in cellular disruption. Also, as the cells move through the valve, they experience decompression and subsequently expand and rupture.

Examples of the application of a French press in laboratory processes may be found on EP1466983B1, EP0842431B1, EP0497771A4, EP3662063A4, among others.

As used herein, the term "biopigment extract" refers to a composition obtained from a microbial culture after cell disruption and at least a filtration step, said composition comprising intracellular pigment molecules released from the microbial cells. The biopigment extract typically comprises one or more pigments together with aqueous medium and minor amounts of soluble cellular components, but is substantially free of intact cells and coarse cellular debris. The extract may be used directly in liquid form, further purified, or subjected to drying or formulation into a powder, paste, or concentrate.

### Results and Discussion

For this study, 9 different strains from 9 different species were cultivated and their biopigments extracted by cell disruption. Importantly, all the referred strains were isolated from the sea (Cabo do Mundo, Matosinhos, Portugal) through the plating of marine sediments, water or algae in Luria Bertrani (10g Tryptone, 5g Yeast Extract, and 10g NaCl, per liter of distilled water), SCN (0 g of soluble starch, 0.3 g of casein, 2 g of K₂HPO₄, 2 g of KNO₃, 2 g of NaCl, 0.05 g of MgSO₄.7H₂O, 0.02 g of CaCO₃, 0.01 g of FeSO₄.7H₂O, and 17 g of agar, per liter of distilled water), Sabouraud Dextrose (40 g dextrose, 10 g peptone, and 15 g agar, per liter of distilled water) and Cetrimide medium (20 g peptone, 1.4 g MgCl₂, 10 g K₂SO₄, 0.3 g cetrimide, and 15 g agar, per liter of distilled water) allowing the isolation of bacteria, actinobacteria, yeast and *Pseudomonas* strains respectively. The referred isolates were identified to the species level using 16S rRNA sanger sequencing, followed by Blastn on NCBI. For the disruption of all referred isolates, 3 cycles of disruption were carried out in the same conditions, as described below.

The strains tested are summarized in **Table 1.**

**Table 1. Strains tested**

| Strain number | Strain |
|---|---|
| Strain 1 | *Candida albicans* |
| Strain 2 | *Streptomyces coelicolor* |
| Strain 3 | *Bacillus subtilis* |
| Strain 4 | *Streptomyces brasiliensis* |
| Strain 5 | *Streptomyces griseus* |
| Strain 6 | *Rhodotorula minuta* |
| Strain 7 | *Bacillus nakamurai* |
| Strain 8 | *Pseudomonas azotoformans* |
| Strain 9 | *Pseudomonas fluorescens* |

The procedure for the cell disruption using a French press was the following: After harvesting the bacterial or yeast cultures, the microbiological culture suspension was subjected to cell disruption using a French press, a mechanical device designed to lyse cells through high-pressure forces. The process begins by passing the microbiological culture through the French press, where it is exposed to high pressure, preferably between 13.7895 and 344.7378 MPa (2,000 and 50,000 psi). For strains 1-9, the pressure applied was 137.8951 MPa (20,000 psi). The microbiological culture suspension, having a volume of 30 mL and an optical density measured at 600 nm between 0.1 and 1.9, was maintained at a temperature of 30 °C. The pH of the microbiological culture suspension ranges from 6-8 in the strains tested. As the suspension is forced through a narrow valve under this pressure, the cells experience intense shear forces and sudden decompression. This combination of mechanical force and rapid decompression effectively ruptures the cell walls and membranes, releasing the biopigments into the surrounding buffer. Following the disruption, the lysate, which contains the released biopigments, was subjected to a first filtration through a first filter, having a pore size of 5 micrometres, to remove cell debris. The resulting filtrate was then subjected to a second filtration through a second filter, having a pore size of 0.45 micrometres, to remove residual fine particles and microbial contaminants, thereby yielding a clarified biopigment extract suitable for downstream purification or direct use.

The Optical Density (OD) was measured before and after each cycle of cell disruption. For this, a volume of each culture before cell disruption was filtered (0.45 µM) to allow a correct OD measurement.

Moreover, the OD was measured in a wavelength range between 380 nm and 740 nm, in order to identify the wavelength with maximum absorption for each strain. By the analysis of the obtained absorption spectrum, it was possible to define maximum absorption for each strain: Strain 1 - 540 nm; Strain 2 - 520 nm; Strain 3 - 440 nm; Strain 4 - 560 nm; Strain 5 - 560 nm; Strain 6 - 500 nm; Strain 7 - 680 nm; Strain 8 - 550 nm; Strain 9 - 520 nm.

In the graphic of **Figure 1****,** it is possible to observe the changes in OD before cell disruption and after each cycle.

As it can be seen in **Figure 1****,** the OD value increased with each cell disruption cycle using the method of the present disclosure, with exception for strains 3, 4 and 6. These strains had smaller aggregates during growth, leading to some solid particles in suspension even after filtering and thus justifying the OD values before disruption. After at least 2 disruption cycles the OD for all strains increased, corroborating our method. The obtained results showed that the cell disruption process combined with a filtration step, can be used to obtain a cleaner pigment and thus increasing dyeing efficiency.

**Table 2** summarizes the OD values obtained for each strain.

**Table 2. OD values for each strain in the three French Press cycles, at the maximum absorption wavelength.**

| **Strain** | **Method of the present disclosure French Press cycles** | | | |
|---|---|---|---|---|
| - | 0 | 1 | 2 | 3 |
| Strain 1 | 1.334 | 1.714 | 1.76 | 1.773 |
| Strain 2 | 1.058 | 1.268 | 1.267 | 1.301 |
| Strain 3 | 0.219 | 0.213 | 0.256 | 0.267 |
| Strain 4 | 0.341 | 0.335 | 0.415 | 0.425 |
| Strain 5 | 0.351 | 0.798 | 0.834 | 0.832 |
| Strain 6 | 0.11 | 0.116 | 0.246 | 0.23 |
| Strain 7 | 0.456 | 1.785 | 1.859 | 1.86 |
| Strain 8 | 1.405 | 1.5 | 1.54 | 1.58 |
| Strain 9 | 0.291 | 0.297 | 0.32 | 0.34 |

Multifiber fabrics, composed of acetate, cotton, nylon, polyester, acrylic and wool were dyed with cell culture before disruption with French press and after using the procedure of the present disclosure, after the 3^{rd} disruption cycle. Afterwards fabrics were washed in a laboratory-scale dyeing machine, and the dying efficiency was observed. This observation was performed through RGB measurements. RGB represents color as a combination of red, green, and blue light intensities ranging from 0 to 255 and is used to quantify and compare colors objectively across samples while allowing digital analysis and standardization. **Table 3** presents the RGB measurements for the two fabrics showing the most pronounced results, using the culture before and after the French press method, for the nine tested strains. In general, higher RGB values correspond to lighter colors while lower RGB values correspond to darker colors. The combination of the red, green, and blue components determines the specific hue, so RGB indicates the brightness of a color rather than its saturation.

**Table 3.** RGB measured for the two fabrics showing the most pronounced results, using the culture before and after the French press method, for the nine tested strains. RGB refers to the contribution of red, green, and blue components that determines the specific hue, thus indicating the brightness of a color.

| | | **Method of the present disclosure (mechanical disruption using a French Press, three disruption cycles)** | | | **Comparative method (Without mechanical disruption using a French press)** | | |
|---|---|---|---|---|---|---|---|
| **Strain** | **Fabric** | **R (red)** | **G (green)** | **B (blue)** | **R (red)** | **G (green)** | **B (blue)** |
| Strain 1 | Nylon | 154 | 150 | 117 | 228 | 218 | 194 |
| | Wool | 195 | 184 | 154 | 216 | 197 | 164 |
| Strain 2 | Nylon | 158 | 129 | 134 | 161 | 152 | 159 |
| | Wool | 146 | 114 | 114 | 154 | 144 | 142 |
| Strain 3 | Nylon | 187 | 170 | 155 | 233 | 225 | 213 |
| | Wool | 179 | 156 | 140 | 228 | 208 | 183 |
| Strain 4 | Nylon | 237 | 180 | 161 | 238 | 238 | 234 |
| | Polyester | 228 | 194 | 221 | 227 | 222 | 213 |
| Strain 5 | Nylon | 227 | 220 | 232 | 238 | 238 | 233 |
| | Wool | 178 | 170 | 168 | 234 | 219 | 200 |
| Strain 6 | Cotton | 241 | 220 | 205 | 224 | 226 | 218 |
| | Acrylic | 242 | 216 | 200 | 240 | 240 | 235 |
| Strain 7 | Cotton | 171 | 165 | 155 | 236 | 232 | 224 |
| | Polyester | 154 | 148 | 151 | 227 | 223 | 215 |
| Strain 8 | Nylon | 189 | 181 | 180 | 216 | 215 | 219 |
| | Polyester | 173 | 175 | 183 | 220 | 221 | 224 |
| Strain 9 | Nylon | 227 | 220 | 232 | 238 | 238 | 233 |
| | Cotton | 234 | 218 | 196 | 246 | 239 | 230 |
| **Average** | | **188** | **178** | **165** | **228** | **223** | **217** |

By analyzing the results presented in **Table 3,** it is possible to verify that the fabrics dyed with the biopigment extract obtained with the method of the present disclosure showed more diversified colors, whilst fabrics dyed with a biopigment extract obtained with different methods (non-disrupted cells) showed very similar RGB values between them, most usually around the beige and light yellow/grey colors. Moreover, the fabrics dyed with the biopigment extract obtained with the method of the present disclosure showed, in general, lower RGB values, corresponding to darker colors.

A relative comparison of the RGB values obtained with the two methods further highlights the improvement achieved by the method of the present disclosure. On average, the fabrics treated with extracts obtained by the method herein described exhibited values of 188 (R), 178 (G) and 165 (B), whereas fabrics treated with extracts from the comparative method (non-disrupted cells) presented higher and less diversified averages of 228 (R), 223 (G) and 217 (B). This corresponds to a relative decrease of approximately 18% in red, 20% in green and 24% in blue values when using the method of the present disclosure. Such a reduction reflects a significant enhancement in color depth and intensity. In particular cases, the relative difference reached up to about 32% for the blue component (Strain 1 - Nylon) and 27% for the red component (Strain 3 - Nylon), evidencing that the disclosed method allows the release and effective use of intracellular pigments otherwise inaccessible with conventional methods.

Thus, the method of the present disclosure allowed the release of pigment that usually would only be available intracellularly and would not contribute for the fabric dyeing. This fact was observed mainly for strains 4, 5, 6 and 7, corroborating the improved method herein described.

### Comparative results

During the development of this method, other disruption technique was performed, however the results were not as good as the ones obtained the method of the present disclosure. The comparative method comprised mechanical lysis via freeze-thaw and grinding and posterior use of beads. This method was tried for strains 7 and 8.

Table 4 shows the RGB measurements for the fabrics submitted to this disruption technique, for the same 2 fabrics presented for the method of the present disclosure (for comparison).

**Table 4. RGB measured for Cotton and Polyester in strain 7 and Nylon and Polyester for strain 8, after dying with cells submitted to mechanical lysis.**

| | | **Comparative method (mechanical lysis via freeze-thaw and grinding and posterior use of beads)** | | |
|---|---|---|---|---|
| **Strain** | **Fabric** | **R (red)** | **G (green)** | **B (blue)** |
| Strain 7 | Cotton | 213 | 210 | 205 |
| | Polyester | 202 | 200 | 201 |
| Strain 8 | Nylon | 208 | 203 | 202 |
| | Polyester | 218 | 219 | 222 |

When comparing the results obtained with standard cell disruption techniques, based on alternative mechanical disruption methods, it is observed that the method of the present disclosure provides superior performance. In particular, the process described herein results in increased yields of the target intracellular pigments, as well as pigments of enhanced and more consistent quality, including improved color intensity, stability, and purity.

It can be concluded that, the method of the present disclosure, in particular combining the cell disruption by French press with a filtration step, was efficient to break the cells and release higher content of biopigments. With this procedure, an increase of the absorbance (at the specific color wavelength) and a more efficient dyeing result, was possible to achieve.

### MATERIALS AND METHODS

### Cultivation of Bacterial and Yeast Cultures

The cultivation of bacterial and yeast cultures was carried out to achieve optimal bioproduct yields. Initially, appropriate strains of bacteria or yeast were selected based on their capability to produce the target bioproducts. A seed culture was prepared under standard conditions to inoculate the larger-scale cultivation. The growth medium was formulated with the necessary nutrients specific to the selected microorganisms. Cultivation was conducted under controlled conditions, with temperature and pH adjusted to the optimal levels for each microorganism. Aeration was provided as needed to support aerobic growth. Throughout the cultivation process, optical density (OD) at 600 nm was regularly measured to monitor culture growth. Cultures were maintained until the OD reached a target range, typically between 0.1 and 2.0, depending on the microorganism and the biopigments being produced. This OD range was chosen to ensure high cell density and maximum production efficiency. Upon reaching the desired OD, cultures were harvested for subsequent bioproduct extraction and processing.

### Optical Density (OD)

The optical density (OD) of the microbiological culture was determined by spectrophotometric measurement in a wavelength range between 380 nm and 740 nm using a standard UV-Vis spectrophotometer microplate reader for optical density measurement. Prior to measurement, the instrument was calibrated against a blank consisting of the corresponding sterile culture medium. All the cultures were measured before any kind of disruption and after each disruption cycle followed by filtration. Culture samples were diluted with sterile medium, where necessary, to obtain values within the linear range of the spectrophotometer.

In certain embodiments, microbial strains including *Streptomyces coelicolor, Bacillus subtilis, Streptomyces brasiliensis, Streptomyces griseus, Rhodotorula minuta, Bacillus nakamurai, Pseudomonas azotoformans,* and *Pseudomonas fluorescens* are capable of producing pigments of industrial relevance. Specifically, *Streptomyces* species and *Bacillus* spp. generate dark, melanin-like pigments when cultivated in the presence of precursors such as L-3,4-dihydroxyphenylalanine (L-DOPA), N-acetylglucosamine (NAG), catechol, or tyrosine, conditions that stimulate oxidative polymerization and secondary metabolism. All these nutrients are present on the SCN media where these strains were isolated (Avetisyan S, Hovsepyan A, Saghatelyan L, Koloyan H, Chizhik O, Hovhannisyan S, Paronyan M. Obtaining Melanin-Synthesizing Strains of Bacillus thuringiensis and their Use for Biological Preparations. Front Biosci (Elite Ed). 2024 Aug 13;16(3):27.; Kraseasintra O, Sensupa S, Mahanil K, Yoosathaporn S, Pekkoh J, Srinuanpan S, Pathom-Aree W, Pumas C. Optimization of Melanin Production by Streptomyces antibioticus NRRL B-1701 Using Arthrospira (Spirulina) platensis Residues Hydrolysates as Low-Cost L-tyrosine Supplement. BioTech (Basel). 2023 Mar 20;12(1):24) *Rhodotorula minuta* predominantly produces red to orange carotenoid pigments, with enhanced yields under carbon-rich and nitrogen-limiting conditions. These conditions are provided by the media Sabouraud Dextrose where this strain was isolated (Karanjgaokar, Diksha R., and Kishori S. Tarfe. Isolation of pigmented yeasts, extraction of pigment and study of antimicrobial property of its pigment. Int J Curr Microbiol Appl Sci 6.7 (2017): 664-672). *Pseudomonas fluorescens* produces fluorescent pigments such as pyoverdine under iron-limiting environments, which are the conditions provided by cetrimide medium where this strain was isolated (Albesa, Inés, et al. Pyoverdine production by Pseudomonas fluorescens in synthetic media with various sources of nitrogen. Microbiology 131.12 (1985): 3251-3254). Moreover, several *Pseudomonas* species are reported to produce Pyocyanin, a blueish pigment, when grown in cetrimide medium. In concrete, *Pseudomonas azotoformans* lacks literature referring its growth on cetrimide medium, however this specific species produces blue pigment when growing on spoilages (Circella, Elena, et al. Pseudomonas azotoformans belonging to Pseudomonas fluorescens group as causative agent of blue coloration in carcasses of slaughterhouse rabbits. Animals 10.2 (2020): 256) Collectively, these organisms provide a diverse portfolio of bio-based pigments ranging from melanins and phenolic polymers to carotenoids and fluorescent siderophores, thereby enabling broad applications in textiles, cosmetics, and biomedical materials.

In certain embodiments, *Candida albicans* were cultivated in sabouraud dextrose media, where it produces melanin or melanin-like pigments, acquiring a cream/brownish pigmentation (Astekar M, Bhatiya PS, Sowmya GV. Prevalence and characterization of opportunistic candidal infections among patients with pulmonary tuberculosis. J Oral Maxillofac Pathol. 2016 May-Aug;20(2):183-9)

### Cell Disruption Using a French Press

After harvesting the bacterial or yeast cultures, the biomass was subjected to cell disruption using a French press, a mechanical device designed to lyse cells through high-pressure forces. The process begins by passing the cell culture through the French press, where it is exposed to high pressure, preferably between 68.9475 MPa and 137.8951 MPa (10000 and 20000 psi). As the suspension is forced through a narrow valve under this pressure, the cells experience intense shear forces and sudden decompression. This combination of mechanical force and rapid decompression effectively ruptures the cell walls and membranes, releasing the intracellular contents into the surrounding buffer. Following the disruption, the lysate, which contains the released intracellular components, is collected for further processing. The efficiency of cell lysis is monitored by observing the appearance of the lysate and measuring the release of specific intracellular markers.

### Primary Filtration

After obtaining the cell lysate from the French press, it undergoes primary filtration to remove large debris such as cell wall fragments and unbroken cells. The lysate is passed through a coarse filtration system equipped with a filter that has a pore size typically ranging from 0.45 to 10 µm. This filtration step is designed to retain larger particles while allowing smaller molecules, including the target bioproducts, to pass through.

The primary filtration process effectively separates the coarse particulate matter from the liquid containing the dissolved bioproducts. This initial filtration is crucial for clarifying the lysate and preparing it for subsequent purification steps. By removing the larger debris, the primary filtration ensures that the following purification processes can operate more efficiently, focusing on smaller contaminants and achieving higher purity of the final bioproducts.

### Secondary Filtration and Clarification

Following the primary filtration, the clarified lysate is subjected to secondary filtration to achieve a higher level of purification. In this step, the lysate is passed through filters with finer pore sizes, typically between 0.2 and 0.45 µm. This finer filtration process is designed to remove any residual fine particulates and smaller impurities that were not captured during the primary filtration. The secondary filtration system used is equipped with membranes or filter cartridges that have precisely controlled pore sizes to ensure the efficient removal of these smaller contaminants. As the lysate flows through these filters, particles larger than the pore size are retained on the filter surface, while the clarified solution, containing the desired bioproducts, passes through.

### Applications

The extract obtained with the method of the present application may be applied in different matrices, including, but not limiting, textile matrices, paper/cellulose matricies, plastic matricies, cosmetic compositions, among others.

### Method evaluation

To verify the efficiency of the process, the OD of the cell-disrupted cultures was measured in a wavelength range between 380 nm and 740 nm and compared with the entire cell culture.

In terms of application, the efficiency of the process was evaluated with a textile dying procedure, where both entire cell culture and cell-disrupted culture were used for dyeing multifibre fabrics followed by washing in a laboratory-scale dyeing machine. For strains 8 and 9, fabric fluorescence was evaluated to prove the method for fluorescent dyes.

### RGB measurements

For the evaluation of the coloration obtained with the biopigment extracts, the color of the dyed textile fabrics was quantified by measuring the RGB (Red, Green, Blue) values of the samples. The measurements were performed using X-Rite spectrophotometer (Newtek) according to ISO 11664 (CIE colorimetry standards) and analyzed with image-processing software (i-color control) to extract average RGB values from representative areas of the textile surface.

The term "comprising" whenever used in this document is intended to indicate the presence of stated features, integers, steps, components, but not to preclude the presence or addition of one or more other features, integers, steps, components or groups thereof.

Where ranges are given, endpoints are included. Furthermore, it is to be understood that unless otherwise indicated or otherwise evident from the context and/or the understanding of one of ordinary skill in the art, values that are expressed as ranges can assume any specific value within the stated ranges in different embodiments of the invention, to the tenth of the unit of the lower limit of the range, unless the context clearly dictates otherwise. It is also to be understood that unless otherwise indicated or otherwise evident from the context and/or the understanding of one of ordinary skill in the art, values expressed as ranges can assume any subrange within the given range, wherein the endpoints of the subrange are expressed to the same degree of accuracy as the tenth of the unit of the lower limit of the range

The disclosure should not be seen in any way restricted to the embodiments described and a person with ordinary skill in the art will foresee many possibilities to modifications thereof. The above-described embodiments are combinable.

The following dependent claims further set out particular embodiments of the disclosure.

## Claims

1. Method for obtaining a biopigment extract from a microorganism, comprising:
providing a microbiological culture suspension;
subjecting the microbiological culture suspension to at least a cycle of mechanical disruption using a French press, at a pressure ranging from 13.7895 - 344.7378 MPa (2,000 to 50,000 psi);
performing a first filtration of the disrupted cell mixture through a first filter,
wherein said first filter has a pore size ranging from 0.45 to 15 micrometers;
optionally performing a second filtration of the filtrate obtained from the first filtration through a second filter, wherein said second filter has a pore size ranging from 0.2 to 0.45 micrometers,
thereby obtaining a biopigment extract.

2. Method according to the previous claim wherein the method is an organic solvent free method.

3. Method according to any of the previous claims wherein the mechanical disruption using a French press is performed at a pressure ranging from 103.4213 - 172.3689 MPa (15,000-25,000 psi); more preferably 137.8951 MPa (20,000 psi).

4. Method according to any of the previous claims wherein the first filter has a pore size of 5 micrometer and/or the second filter has a pore size of 0.45 micrometers.

5. Method according to any of the previous claims wherein the mechanical disruption is performed at a temperature ranging from 0 °C to 40 °C; preferably 20 °C to 30 °C.

6. Method according to any of the previous claims wherein the microbiological culture suspension has a volume ranging from 10 mL -100 L; preferably 20-40 mL.

7. Method according to any of the previous claims wherein the microbiological culture suspension has an optical density at 600 nm (OD600) ranging from 0.1 - 20; preferably 0.1-4; more preferably 0.5-2.

8. Method according to any of the previous claims wherein the microorganism is selected from a microorganism capable of producing intracellular pigments selected from the list consisting of: bacteria, microalgae, fungi, yeast, or mixtures thereof;

9. Method according to any of the previous claims wherein the microorganism is selected from the group consisting of *Pseudomonas spp., Streptomyces spp., Candida spp, Bacillus spp,* or mixtures thereof.

10. Method according to any of the previous claims wherein the microorganism is selected from the group consisting of: *Candida albicans, Streptomyces coelicolor, Bacillus subtilis, Streptomyces brasiliensis, Streptomyces griseus, Rhodotorula minuta, Bacillus nakamurai, Pseudomonas azotoformans, Pseudomonas fluorescens,* or mixtures thereof.

11. Method according to any of the previous claims wherein the mechanical disruption is performed for two or more mechanical disruption cycles; preferably for three or more mechanical disruption cycles; more preferably 3-4 cycles of mechanical disruption.

12. Biopigment extract obtainable by the method according to any of the previous claims.

13. Fabric or textile material comprising the biopigment extract according to the previous claim 12; preferably the fabric or textile material is selected from the list consisting of: acetate, cotton, wool, nylon, polyester, acrylic, or mixtures thereof.

14. Cosmetic composition comprising the biopigment extract according to the previous claim 12.

15. Apparatus for obtaining a biopigment extract from a microorganism, comprising:
a mechanical cell disruption unit equipped with a French press capable of applying a pressure ranging from 13.7895- 344.7378 MPa (2,000 to 50,000 psi) to a microbial cell suspension;
a first filtration unit configured to receive the disrupted cell mixture from the mechanical cell disruption unit, said first filtration unit comprising a filter with a pore size ranging from 0.45 to 15 micrometers;
optionally, a second filtration unit configured to receive the filtrate from the first filtration unit, said second filtration unit comprising a filter with a pore size ranging from 0.2 to 0.45 micrometers;
a collection unit for receiving the biopigment extract obtained from the first filtration unit or from the second filtration unit.
